# EUROPEAN PATENT SPECIFICATION

(11) **EP 1 961 770 B1**
(45) Date of publication and mention of the grant of the patent: **23.04.2014**
(21) Application number: 08250586.8
(22) Date of filing: 20.02.2008
(51) Int. Cl.: C08B 37/00

(54) **Method of making a complex**
Verfahren zur Herstellung eines Komplexes
Procédé pour la fabrication d'un complexe

(30) Priority: 22.02.2007 US 902731 P
(43) Date of publication of application: 27.08.2008
(73) Proprietor: Rohm and Haas Company, Philadelphia, PA 19106-2399 (US)
(72) Inventor: Jacobson, Richard Martin, Chalfont Pennsylvania 18914 (US); Lau, Willie, Lower Gwynedd Pennsylvania 19002 (US)
(74) Representative: Buckley, Guy Julian

(56) References cited:
- US-A- 3 425 910
- US-A- 3 640 847
- US-A- 3 652 398
- US-A- 4 738 923
- US-A- 6 017 849

## Description

### BACKGROUND:

It is often desired to make a complex that contains a complexing agent ana a useful complexant. Such complexes are useful, for example, for controlling the location of useful complexant. For example, a useful complexant may be maintained as part of the complex until it is desired to release the useful complexant for some purpose.

In the past, complexing agents have been manufactured and purified prior to formation of a complex with a useful complexant. Such purification has been accomplished by various methods, involving various steps, prior to the use of the complexing agent in forming a complex with a useful complexant. For example, G. Schmid, in Comprehensive Supramolecular Chemistry, vol. 3, pp. 41-56 (1966), teaches methods by which cyclodextrins, which are useful as complexing agents, may be manufactured and purified. Some of the manufacturing methods taught by Schmid involve formation of a complex consisting of a cyclodextrin and a manufacturing complexant such as, for example, ethanol, decanol, or cyclohexane, followed by purification steps, including, for example, decomplexation to remove the manufacturing complexant. Other known purification steps include, for example, separation of solid complexing agent (alone or as part of a complex) from a fluid medium, crystallization, drying, other purification steps, and combinations thereof.

In the past, after the complexing agent was manufactured and purified, it was mixed with a useful complexant to form a complex. For example, US 6,017,849 teaches making cyclodextrin, then purifying the cyclodextrin, and then using the purified cyclodextrin to form a complex with cyclopropene and its derivatives.

Previously-known manufacturing methods involved formation of a complex that contained complexing agent and manufacturing complexant. Such a complex was not considered to be useful for any purpose other than as an intermediate material in the process of manufacturing a purified complexing agent. It was considered that, before the complexing agent could be put to use, the manufacturing complexant needed to be removed from the complex. Such manufacturing methods involved, in addition to removal of manufacturing complexant, various steps performed for the purpose of producing purified complexing agent.

It is desired to provide an improved method of making complexes that contain a complexing agent and a useful complexant, in which the improved method omits one or more of the steps from the previously-known manufacturing and/or purification process for making such complexes. For example, it is desired to provide a method of making complexes that contain a complexing agent and a useful complexant, in which the method omits purification of the complexing agent. For example, it is desired to provide a method of manufacturing complexes containing a complexing agent and a useful complexant, in which the method omits one or more of the following steps: removal of manufacturing complexant, separation of solid complexing agent from a fluid medium, separation of a solid complex containing complexing agent and manufacturing complexant from a fluid medium, crystallization of complexing agent, drying of complexing agent, other steps for purifying complexing agent, and combinations thereof.

### STATEMENT OF THE INVENTION:

The present invention, in its various aspects, is as set out in the accompanying claims.
In a first aspect of the present invention, there is provided a method of making at least one inclusion complex comprising a useful complexant and a cyclodextrin, wherein said method comprises the steps of
   (a) making said cyclodextrin, and
   (b) mixing said cyclodextrin with said useful complexant,
wherein said method is conducted without purifying said cyclodextrin, and wherein said useful complexant comprises at least one cyclopropene.

In a second aspect of the present invention, there is provided a composition useful for manufacturing inclusion complexes, wherein said composition comprises water, at least one starch, at least one enzyme, and at least one useful complexant, wherein said useful complexant comprises at least one cyclopropene, wherein said starch and said enzyme are capable of forming a cyclodextrin that is capable of forming an inclusion complex that comprises said cyclodextrin and said useful complexant.

### DETAILED DESCRIPTION:

The present invention is a method of making an inclusion complex or a mixture of inclusion complexes. An inclusion complex is a composition that contains a complexing agent and a complexant. A complexing agent is a compound that provides a cavity, and a complexant is a compound whose molecule is partially or totally located within that cavity.

A complexant participates in an inclusion complex because all of the complexant molecule or a portion of the complexant molecule fits into the cavity provided by the complexing agent. There is no covalent bond between the complexing agent and the complexant. In some cases, neither the complexant nor the complexing agent contains a moiety with a full ionic charge, though one or both of the complexant and the complexing agent may contain within its molecule one or more covalently bound polar groups. A complexant may be held in place in the inclusion compound by, for example, any one of or any combination of the following forces acting between the complexant and the complexing agent: physical fit of the molecules, hydrophobic interactions, dipole interactions (including, for example, hydrogen bonding), induced dipole interactions, van der Waals forces, and other forces.

The complexing agents used in the practice of the present invention are cyclodextrins. Cyclodextrins are compounds whose molecules are cone-shaped structures that have structures that are made from 6 or more glucose units. As used herein, a statement that a cyclodextrin is made from certain glucose units is to be understood as a description of the structure of the cyclodextrin molecule, which may or may not be actually made by reacting those certain glucose molecules. Cyclodextrins may be made from as many as 32 glucose units. Cyclodextrins that are made from 6, 7, and 8 glucose units are known, respectively, as alpha-cyclodextrin, beta-cyclodextrin, and gamma-cyclodextrin. In some embodiments, exactly one of alpha-cyclodextrin, beta-cyclodextrin, or gamma-cyclodextrin is used. In some embodiments, a mixture of two or more of alpha-cyclodextrin, beta-cyclodextrin, and gamma-cyclodextrin is used. Independently, in some embodiments, no complexing agent other than alpha-cyclodextrin is used.

Independent of the number of glucose units in the cyclodextrin, the class of compounds called "cyclodextrins" is considered herein to also include derivatives of cylcodextrin molecules. That is, the term "cyclodextrin" applies herein to molecules that are cone-shaped structures that have structures that are made from 6 or more glucose units and also applies to derivatives of such molecules, when such derivatives are capable of performing as complexing agents. Some suitable derivatives are, for example, molecules that have a structure that is (or could be) formed by addition of an akyl group (such as, for example, a methyl group) to a cyclodextrin. Some other derivatives are, for example, molecules that have a structure that is (or could be) formed by addition of a hydroxyakyl group (such as, for example, a hydroxypropyl group) to a cyclodextrin. Some derivatives that are considered "cylcodextrins" are, for example, methyl-beta-cyclodextrin and hydroxypropyl-alpha-cyclodextrin.

Complexants used in the present invention comprise at least one cyclopropene. As used herein, "a cyclopropene" is any compound with the formula 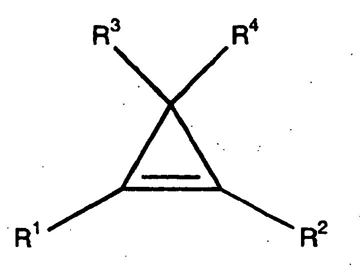 where each R¹, R², R³ and R⁴ is independently selected from the group consisting of H and a chemical group of the formula:

-(L)ₙ-Z

where n is an integer from 0 to 12. Each L is a bivalent radical. Suitable L groups include, for example, radicals containing one or more atoms selected from H, B, C, N, O, P, S, Si, or mixtures thereof. The atoms within an L group may be connected to each other by single bonds, double bonds, triple bonds, or mixtures thereof. Each L group may be linear, branched, cyclic, or a combination thereof. In any one R group (i.e., any one of R¹, R², R³ and R⁴) the total number of heteroatoms (i.e., atoms that are neither H nor C) is from 0 to 6. Independently, in any one R group the total number of non-hydrogen atoms is 50 or less. Each Z is a monovalent radical. Each Z is independently selected from the group consisting of hydrogen, halo, cyano, nitro, nitroso, azido, chlorate, bromate, iodate, isocyanato, isocyanido, isothiocyanato, pentafluorothio, and a chemical group G, wherein G is a 3 to 14 membered ring system.

Among embodiments in which at least one of R¹, R², R³, and R⁴ is not hydrogen and has more than one L group, the L groups within that particular R¹, R², R³, or R⁴ group may be the same as the other L groups within that same R¹, R², R³, or R⁴ group, or any number of L groups within that particular R¹, R², R³, or R⁴ group may be different from the other L groups within that same R¹, R², R³, or R⁴ group.

Among embodiments in which at least one of R¹, R², R³, and R⁴ contains more than one Z group, the Z groups within that R¹, R², R³, or R⁴ group may be the same as the other Z groups within that R¹, R², R³, or R⁴ group, or any number of Z groups within that R¹, R², R³, or R⁴ group may be different from the other Z groups within that R¹, R², R³, or R⁴ group.

The R¹, R², R³, and R⁴ groups are independently selected from the suitable groups. The R¹, R², R³, and R⁴ groups may be the same as each other, or any number of them may be different from the others. Among the groups that are suitable for use as one or more of R¹, R², R³, and R⁴ are, for example, aliphatic groups, aliphatic-oxy groups, alkylphosphonato groups, cycloaliphatic groups, cycloalkylsulfonyl groups, cycloalkylamino groups, heterocyclic groups, aryl groups, heteroaryl groups, halogens, silyl groups, other groups, and mixtures and combinations thereof. Groups that are suitable for use as one or more of R¹, R², R³, and R⁴ may be substituted or unsubstituted. Independently, groups that are suitable for use as one or more of R¹, R², R³, and R⁴ may be connected directly to the cyclopropene ring or may be connected to the cyclopropene ring through an intervening group such as, for example, a heteroatom-containing group.

Among the suitable R¹, R², R³, and R⁴ groups are, for example, aliphatic groups. Some suitable aliphatic groups include, for example, alkyl, alkenyl, and alkynyl groups. Suitable aliphatic groups may be linear, branched, cyclic, or a combination thereof. Independently, suitable aliphatic groups may be substituted or unsubstituted.

As used herein, a chemical group of interest is said to be "substituted" if one or more hydrogen atoms of the chemical group of interest is replaced by a substituent. It is contemplated that such substituted groups may be made by any method, including but not limited to making the unsubstituted form of the chemical group of interest and then performing a substitution. Suitable substituents include, for example, alkyl, alkenyl, acetylamino, alkoxy, alkoxyalkoxy, alkoxycarbonyl, alkoxyimio, carboxy, halo, haloalkoxy, hydroxy, alkylsulfonyl, alkylthio, trialkylsilyl, dialkylamino, and combinations thereof. An additional suitable substituent, which, if present, may be present alone or in combination with another suitable substituent, is

-(L)ₘ-Z

where m is 0 to 8, and where L and Z are defined herein above. If more than one substituent is present on a single chemical group of interest, each substituent may replace a different hydrogen atom, or one substituent may be attached to another substituent, which in turn is attached to the chemical group of interest, or a combination thereof.

Among the suitable R¹, R², R³, and R⁴ groups are, for example, substituted and unsubstituted aliphatic-oxy groups, such as, for example, alkenoxy, alkoxy, alkynoxy, and alkoxycarbonyloxy.

Also among the suitable R¹, R², R³, and R⁴ groups are, for example, substituted and unsubstituted alkylphosphonato, substituted and unsubstituted alkylphosphato, substituted and unsubstituted alkylamino, substituted and unsubstituted alkylsulfonyl, substituted and unsubstituted alkylcarbonyl, and substituted and unsubstituted alkylaminosulfonyl, including, for example, alkylphosphonato, dialkylphosphato, dialkylthiophosphato, dialkylamino, alkylcarbonyl, and dialkylaminosulfonyl.

Also among the suitable R¹, R², R³, and R⁴ groups are, for example, substituted and unsubstituted cycloalkylsulfonyl groups and cycloalkylamino groups, such as, for example, dicycloalkylaminosulfonyl and dicycloalkylamino.

Also among the suitable R¹, R², R³, and R⁴ groups are, for example, substituted and unsubstituted heterocyclyl groups (i.e., aromatic or non-aromatic cyclic groups with at least one heteroatom in the ring).

Also among the suitable R¹, R², R³, and R⁴ groups are, for example, substituted and unsubstituted heterocyclyl groups that are connected to the cyclopropene compound through an intervening oxy group, amino group, carbonyl group, or sulfonyl group; examples of such R¹, R², R³, and R⁴ groups are heterocyclyloxy, heterocyclylcarbonyl, diheterocyclylamino, and diheterocyclylaminosulfonyl.

Also among the suitable R¹, R², R³, and R⁴ groups are, for example, substituted and unsubstituted aryl groups. Suitable substituents are those described herein above. In some embodiments, one or more substituted aryl group is used in which at least one substituent is one or more of alkenyl, alkyl, alkynyl, acetylamino, alkoxyalkoxy, alkoxy, alkoxycarbonyl, carbonyl, alkylcarbonyloxy, carboxy, arylamino, haloalkoxy, halo, hydroxy, trialkylsilyl, dialkylamino, alkylsulfonyl, sulfonylalkyl, alkylthio, thioalkyl, arylaminosulfonyl, and haloalkylthio.

Also among the suitable R¹, R², R³, and R⁴ groups are, for example, substituted and unsubstituted heterocyclic groups that are connected to the cyclopropene compound through an intervening oxy group, amino group, carbonyl group, sulfonyl group, thioalkyl group, or aminosulfonyl group; examples of such R¹, R², R³, and R⁴ groups are diheteroarylamino, heteroarylthioalkyl, and diheteroarylaminosulfonyl.

Also among the suitable R¹, R², R³, and R⁴ groups are, for example, hydrogen, fluoro, chloro, bromo, iodo, cyano, nitro, nitroso, azido, chlorato, bromato, iodato, isocyanato, isocyanido, isothiocyanato, pentafluorothio; acetoxy, carboethoxy, cyanato, nitrato, nitrito, perchlorato, allenyl; butylmercapto, diethylphosphonato, dimethylphenylsilyl, isoquinolyl, mercapto, naphthyl, phenoxy, phenyl, piperidino, pyridyl, quinolyl, triethylsilyl, trimethylsilyl; and substituted analogs thereof.

As used herein, the chemical group G is a 3 to 14 membered ring system. Ring systems suitable as chemical group G may be substituted or unsubstituted; they may be aromatic (including, for example, phenyl and napthyl) or aliphatic (including unsaturated aliphatic, partially saturated aliphatic, or saturated aliphatic); and they may be carbocyclic or heterocyclic. Among heterocyclic G groups, some suitable heteroatoms are, for example, nitrogen, sulfur, oxygen, and combinations thereof. Ring sysytems suitable as chemical group G may be monocyclic, bicyclic, tricyclic, polycyclic, spiro, or fused; among suitable chemical group G ring systems that are bicyclic, tricyclic, or fused, the various rings in a single chemical group G may be all the same type or may be of two or more types (for example, an aromatic ring may be fused with an aliphatic ring).

In some embodiments, G is a ring system that contains a saturated or unsaturated 3 membered ring, such as, for example, a substituted or unsubstituted cyclopropane, cyclopropene, epoxide, or aziridine ring.

In some embodiments, G is a ring system that contains a 4 membered heterocyclic ring; in some of such embodiments, the heterocyclic ring contains exactly one heteroatom. Independently, in some embodiments, G is a ring system that contains a heterocyclic ring with 5 or more members; in some of such embodiments, the heterocyclic ring contains 1 to 4 heteroatoms. Independently, in some embodiments, the ring in G is unsubstituted; in other embodiments, the ring system contains 1 to 5 substituents; in some of the embodiments in which G contains substituents, each substituent is independently chosen from the substituents described herein above. Also suitable are embodiments in which G is a carbocyclic ring system.

In some embodiments, each G is independently a substituted or unsubstituted phenyl, pyridyl, cyclohexyl, cyclopentyl, cycloheptyl, pyrolyl, furyl, thiophenyl, triazolyl, pyrazolyl, 1,3-dioxolanyl, or morpholinyl. Among these embodiments include those embodiments, for example, in which G is unsubstituted or substituted phenyl, cyclopentyl, cycloheptyl, or cyclohexyl. In some of these embodiments, G is cyclopentyl, cycloheptyl, cyclohexyl, phenyl, or substituted phenyl. Among embodiments in which G is substituted phenyl are embodiments, for example, in which there are 1, 2, or 3 substituents. Independently, also among embodiments in which G is substituted phenyl are embodiments, for example, in which the substituents are independently selected from methyl, methoxy, and halo.

Also contemplated are embodiments in which R³ and R⁴ are combined into a single group, which is attached to the number 3 carbon atom of the cyclopropene ring by a double bond. Some of such compounds are described in US Patent Publication 2005/0288189.

In some embodiments, one or more cyclopropenes are used in which one or more of R¹, R², R³, and R⁴ is hydrogen. In some embodiments, R¹ or R² or both R¹ and R² is hydrogen. Independently, in some embodiments, R³ or R⁴ or both R³ and R⁴ is hydrogen. In some embodiments, R², R³, and R⁴ are hydrogen.

In some embodiments, one or more of R¹, R², R³, and R⁴ is a structure that has no double bond. Independently, in some embodiments, one or more of R¹, R², R³, and R⁴ is a structure that has no triple bond. Independently, in some embodiments, one or more of R¹, R², R³, and R⁴ is a structure that has no halogen atom substituent. Independently, in some embodiments, one or more of R¹, R², R³, and R⁴ is a structure that has no substituent that is ionic.

In some embodiments, one or more of R¹, R², R³, and R⁴ is hydrogen or (C₁-C₁₀) alkyl. In some embodiments, each of R¹, R², R³, and R⁴ is hydrogen or (C₁-C₈) alkyl. In some embodiments, each of R¹, R², R³, and R⁴ is hydrogen or (C₁-C₄) alkyl. In some embodiments, each of R¹, R², R³, and R⁴ is hydrogen or methyl. In some embodiments, R¹ is (C₁-C₄) alkyl and each of R², R³, and R⁴ is hydrogen. In some embodiments, R¹ is methyl and each of R², R³, and R⁴ is hydrogen, and the cyclopropene is known herein as "1-MCP."

The cyclopropenes applicable to this invention may be prepared by any method. Some suitable methods of preparation of cyclopropenes are the processes disclosed in U.S. Patents No. 5,518,988 and 6,017,849 and in US Patent Publication 2005/0065033.

In some embodiments (herein called "solution embodiments"), a cyclodextrin is made by a chemical reaction that takes place in solution (i.e., one or more reactant is dissolved in a solvent). In some solution embodiments, the cyclodextrin also is a solute in the same solution. Independently, in some solution embodiments, the chemical reaction takes place in an aqueous solution. An aqueous solution is a solution in which the solvent is 50% or more water by weight, based on the weight of the solvent. In some aqueous solutions, the amount of water, by weight, based on the weight of the solvent, is 75% or more; or 90% or more; or 95% or more.

In the practice of the present invention, the cyclodextrin is mixed with a useful complexant. It is contemplated that, in some embodiments, one or more useful complexant may be present in the same container in which the cyclodextrin is being made, while the cyclodextrin is being made. Also contemplated are embodiments in which one or more useful complexant is added to the container after the cyclodextrin is made. Further contemplated are combinations thereof, such as, for example, embodiments in which some useful complexant is present while the cyclodextrin is being made and also some useful complexant is added after the cyclodextrin is made. Additionally contemplated are other variations of such methods, including, for example, embodiments in which one or more of the ingredients needed to make the cyclodextrin (such as, for example, one or more reactant and/or one or more catalyst) are added to a container simultaneously with some or all of a useful complexant.

In the practice of the present invention, when a cyclodextrin and a useful complexant are mixed, an inclusion complex of the present invention is formed. It is contemplated that, in some embodiments, there may be some of the molecules of cyclodextrin that do not become part of an inclusion complex, even if some molecules of useful complexant are present that are likewise not part of an inclusion complex. Also, it is contemplated that, in some embodiments, there may be some of the molecules of useful complexant that do not become part of an inclusion complex, even if some molecules of cyclodextrin are present that are likewise not part of an inclusion complex.

In the practice of previously-known methods, procedures were performed for purification of the cyclodextrin. That is, such methods involved production of purified cyclodextrin that was not part of an inclusion complex. Procedures for purification of the cyclodextrin are any procedures that are performed in order to separate the cyclodextrin from other materials that are present in the same container with the cyclodextrin when the cyclodextrin is made. Removal of small amounts of solvent is not considered purification of cyclodextrin.

One known procedure in previously-known methods that is considered a procedure for purification of cyclodextrin is the procedure of forming a manufacturing complex, which is an inclusion complex that contains the cyclodextrin and a manufacturing complexant. A manufacturing complexant is a complexant that is used for the purpose of making and/or purifying a cyclodextrin. A manufacturing complexant is included in a manufacturing complex, and the manufacturing complexant is later removed. The removal of manufacturing complexant is performed as part of a procedure for purifying cyclodextrin; the removal of manufacturing complexant is not performed in order to put the manufacturing complexant to some use other than manufacture of cyclodextrin.

Some manufacturing complexants are, for example, cyclohexane or other unsubstituted cycloalkanes. Some other examples of manufacturing complexants are unsubstituted alkanes. Some other examples of manufacturing complexants are tetrachloroethane, trichloroethylene, and mixtures thereof. Some other examples of manufacturing complexants are bromobenzene. Some other examples of manufacturing complexants are halogenated hydrocarbons. Some other examples of manufacturing complexants are benzene ,toluene, and alpha-naphthol. Some other examples of manufacturing complexants are aryl compounds and alkaryl compounds. Some other examples of manufacturing complexants are ethanol, 1-butanol, 1-octanol, and 1-decanol. Some other examples of manufacturing complexants are hydroxy-substituted alkanes. Some other examples of manufacturing complexants are sodium lauryl sulfate. Some other examples of manufacturing complexants are anionic surfactants. Some other examples of manufacturing complexants are surfactants. Some other examples of manufacturing complexants are ketones with 3 or 4 carbon atoms. Some other examples of manufacturing complexants are ketones. Another example of a manufacturing complexant is carbon disulfide. Also, mixtures of manufacturing complexants are also considered manufacturing complexants.

Another procedure that is considered a procedure for purification of cyclodextrin is the procedure of first making a manufacturing complex and then (possibly after intervening steps) removing the manufacturing complexant. Intervening steps, if used, may include, for example, any one or combination of the following: separation of a manufacturing complex from solvent (for example, by precipitating or allowing to precipitate); washing of a manufacturing complex; drying of a manufacturing complex; or crystallization of a manufacturing complex. Each of such intervening steps, and any combination of such intervening steps, is considered a procedure for purification of cyclodextrin.

Also considered a procedure for purification of cyclodextrin is removal of manufacturing complexant from a manufacturing complex. One such procedure, for example, is using steam to extract the manufacturing complexant from the manufacturing complex.

Independently, some procedures that are considered to be procedures for purification of cyclodextrin include, for example, procedures that increase the relative concentration of pure cyclodextrin (i.e., cyclodextrin that is not part of an inclusion complex). Such procedures include, for example, any one or combination of the following: separation of pure cyclodextrin from solvent; drying of pure cyclodextrin; distillation of pure cyclodextrin; or crystallization of pure cyclodextrin.

In some embodiments of the present invention, a preliminary mixture is present that contains water, at least one starch, at least one enzyme, and at least one useful complexant. At least one starch and at least one enzyme in such a preliminary mixture are capable of reacting to form a cyclodextrin that is capable of forming an inclusion complex with at least one useful complexant in the preliminary mixture. The preliminary mixture may or may not include cyclodextrin produced by the reaction between the starch and the enzyme. Independently, the preliminary mixture may or may not include one or more additional ingredients.

In the practice of the present invention, "making" the cyclodextrin is defined herein as the chemical reaction that involves starch molecules and enzyme and that forms the cone-shaped structure of the cyclodextrin molecules. Other chemical reactions that may optionally be performed that involve a cyclodextrin molecule but that do not alter the cone-shaped structure are not considered herein to be "making" the cyclodextrin. For example, a process that starts with a cyclodextrin molecule and involves adding a pendant chemical group to that cyclodextrin molecule to form a modified cyclodextrin molecule is not considered herein to be a process of "making" the modified cyclodextrin.

For example, a process that falls outside of the present invention would be the following. A cyclodextrin is made by reacting starch and enzyme, and the resulting cyclodextrin is then purified. Then, that cyclodextrin is subjected to a chemical reaction that adds a methyl group to form a methyl-cyclodextrin, which is then (without additional purification) mixed with a useful complexant to form an inclusion complex. Such a process is considered herein to fall outside the present invention because the cyclodextrin is purified after it is made (i.e., after its cone-shaped structure is formed).

A method is considered to be conducted without purifying the cyclodextrin if it omits any one procedure for purification of cyclodextrin, or if the method omits any combination of procedures for purification of cyclodextrin. Also contemplated are methods of the present invention that are conducted without any procedure for purification of cyclodextrin.

For example, some methods of the present invention omit forming an inclusion complex containing a cyclodextrin and a manufacturing complexant. Independently, some methods of the present invention omit removing a manufacturing complexant from an inclusion complex. For example, some methods of the present invention omit subjecting a manufacturing complex to boiling water. For example, some methods of the present invention omit exposing a manufacturing complex to steam under conditions that remove manufacturing complexant. For example, some methods of the present invention omit subjecting manufacturing complex to solvent extraction to remove manufacturing complexant. Also contemplated are methods of the present invention that omit any combination of the above steps.

Independently, in some embodiments of the present invention, the method performed omits subjecting a cyclodextrin to the following sequence of steps: (A) formation of a composition that contains an inclusion complex with that cyclodextrin and a first complexant, (B) followed by systematic removal of first complexant from that composition, (C) followed by formation of a new composition using that cyclodextrin, where the new composition contains a new inclusion complex with that cyclodextrin and a second complexant. It is contemplated that, in some embodiments of the present invention, the sequence of steps (A), (B), and (C) are not performed, even if other steps are performed before steps (A), (B), and (C); in between any pair of (A), (B), and (C); after (A), (B), and (C); or any combination thereof.

Independently, some methods of the present invention omit separating cyclodextrin that is not contained in an inclusion complex from solvent. Independently, some methods of the present invention omit washing a cyclodextrin that is not contained in an inclusion complex. Independently, some methods of the present invention omit drying a cyclodextrin that is not contained in an inclusion complex. Also contemplated are methods of the present invention that omit any combination of the above steps.

In some solution embodiments of the present invention, the inclusion complex is soluble in the solvent. In some of such embodiments, the solution in which the inclusion complex is dissolved may be used as it is (i.e., without purification) for subsequent purposes.

Independently, in some solution embodiments in which the inclusion complex of the present invention is soluble in the solvent, the solution in which the inclusion complex is dissolved may be subjected to one or more purification processes to make a composition in which the inclusion complex is present in higher concentration than its concentration in the solution. For example, the inclusion complex may be removed and, optionally, dried; or some materials other than the inclusion complex may be removed from the solution; or some solvent may be removed, for example by evaporation; or a combination thereof may be performed.

Also contemplated are solution embodiments in which the inclusion complex of the present invention is poorly soluble in the solvent. That is, a poorly soluble inclusion complex has solubility in the solvent at 25°C, per 100 grams of solvent, 5 gram or less, or 1 gram or less; or 0.3 gram or less; or 0.1 gram or less; or 0.03 gram or less; or 0.01 gram or less.

In some cyclodextrin embodiments of the present invention, the cyclodextrin is made by a chemical reaction involving starch (optionally pre-liquified) and a cyclodextrin-producing enzyme. One known cyclodextrin-producing enzyme, for example, is cyclodextrin glycosyltransferase ("CGTase").

Starch that is suitable for making cyclodextrin includes, for example, modified or unmodified starch derived from cereal or tuber origin, and the amylose or amylopectin fractions thereof. Suitable starch may be, for example, produced from corn, potato, waxy maize, other plants, or a mixture thereof.

In some embodiments, the starch and the cyclodextrin-producing enzyme are dissolved in aqueous solution, where they interact to form cyclodextrin. In some of such embodiments, the inclusion complex that forms when the cyclodextrin interacts with a useful complexant remains soluble in the aqueous solution.

In some cyclodextrin embodiments (herein called "CD-P embodiments"), the starch and the cyclodextrin-producing enzyme are dissolved in aqueous solution when they interact to form cyclodextrin, and the inclusion complex that forms when the cyclodextrin interacts with a useful complexant has poor solubility in the aqueous solution and precipitates. It is contemplated that in the practice of CD-P embodiments, such an inclusion complex will be allowed to precipitate. In some CD-P embodiments, the precipitation of the inclusion complex is desirable because removing the cyclodextrin product via precipitation of the inclusion complex drives the reaction toward producing further cyclodextrin, thereby increasing the yield of cyclodextrin. In some CD-P embodiments, the interaction of enzyme with starch is capable of producing a mixture of cyclodextrins (for example, a mixture of alpha-, beta-, and gamma-cyclodextrins). If a useful complexant is used that tends to form an inclusion complex with a particular one of those cyclodextrins more than with the other cyclodextrins, the precipitated inclusion complex will be rich in complexes that contain that particular cyclodextrin. Also contemplated are CD-P embodiments in which a useful complexant is used that have no tendency to form an inclusion complex with one of the cyclodextrins that are present over other present cyclodextrins that are present.

Independently, in some CD-P embodiments, the precipitated inclusion complex is isolated from the aqueous solution and, optionally, washed, sometimes with water, and optionally dried.

Also contemplated are cyclodextrin embodiments ("CD-S" embodiments) in which the starch and the cyclodextrin-producing enzyme are dissolved in aqueous solution, where they interact to form cyclodextrin, and the inclusion complex that forms when the cyclodextrin interacts with a useful complexant has good solubility in the aqueous solution. Like the CD-P embodiments, in some CD-S embodiments the production of soluble inclusion complex helps to drive the reaction that produces cyclodextrin. CD-S embodiments are contemplated that tend to enhance production of one type of cyclodextrin in preference to other types of cyclodextrin. Also contemplated are CD-S embodiments that do not enhance production of one type of cyclodextrin over other types of cyclodextrin.

In some embodiments, the method of the present invention includes one or more additional optional steps. For example, some cyclodextrin embodiments include liquification of starch. That is, in some cyclodextrin embodiments involving starch as one ingredient and cyclodextrin-producing enzyme as another ingredient, the starch is liquified prior to being mixed with cyclodextrin-producing enzyme. Starch is liquified either by heat, mechanical treatment, acid treatment, exposure to one or more enzyme that liquefies starch, or a combination thereof. Liquification of starch is sometimes a partial hydrolysis process. In some embodiments, liquification of starch is performed by mixing starch with one or more alpha-amylase. In some embodiments, starch, prior to liquification, is chosen that is high in amylopectin. In some embodiments, liquification of starch is performed in aqueous solution, and then, in some of such embodiments, cyclodextrin-producing enzyme and useful complexant are added (sequentially in any order or simultaneously or a combination thereof) to the same aqueous solution.

In the practice of the present invention, it is contemplated that the ingredients for making the complexing agent are chosen to be appropriate for use with the intended useful complexant. That is, if it is intended to use a particular useful complexant, and it is known that that particular useful complexant readily forms inclusion complex with a particular complexing agent, it is contemplated that ingredients will be chosen that are known to yield that particular complexing agent.

When it is stated herein that "no appreciable amount" of a certain inclusion complex is used, it is meant that the molar ratio of that certain inclusion complex to inclusion complexes that fall within the present invention is 0.1 or less; or 0.01 or less; or 0.001 or less; or zero.

Embodiments are contemplated in which no appreciable amount of inclusion complex is made in which the complexant is a complexant other that a useful complexant.

Independently, embodiments are contemplated in which no appreciable amount of inclusion complex is made in which the complexant is cyclohexane. Independently, embodiments are contemplated in which no appreciable amount of inclusion complex is made in which the complexant is an unsubstituted cycloalkane. Independently, embodiments are contemplated in which no appreciable amount of inclusion complex is made in which the complexant is an unsubstituted alkane.

Independently, embodiments are contemplated in which no appreciable amount of inclusion complex is made in which the complexant is tetrachlorethane or trichloroethylene or a mixture thereof. Independently, embodiments are contemplated in which no appreciable amount of inclusion complex is made in which the complexant is bromobenzene. Independently, embodiments are contemplated in which no appreciable amount of inclusion complex is made in which the complexant is a halogenated hydrocarbon.

Independently, embodiments are contemplated in which no appreciable amount of inclusion complex is made in which the complexant is benzene. Independently, embodiments are contemplated in which no appreciable amount of inclusion complex is made in which the complexant is toluene. Independently, embodiments are contemplated in which no appreciable amount of inclusion complex is made in which the complexant is alpha-napthol. Independently, embodiments are contemplated in which no appreciable amount of inclusion complex is made in which the complexant is an aryl compound or an alkaryl compound or a mixture thereof.

Independently, embodiments are contemplated in which no appreciable amount of inclusion complex is made in which the complexant is ethanol. Independently, embodiments are contemplated in which no appreciable amount of inclusion complex is made in which the complexant is 1-butanol. Independently, embodiments are contemplated in which no appreciable amount of inclusion complex is made in which the complexant is 1-octanol. Independently, embodiments are contemplated in which no appreciable amount of inclusion complex is made in which the complexant is 1-decanol. Independently, embodiments are contemplated in which no appreciable amount of inclusion complex is made in which the complexant is a hydroxy-substituted alkane.

Independently, embodiments are contemplated in which no appreciable amount of inclusion complex is made in which the complexant is sodium lauryl sulfate. Independently, embodiments are contemplated in which no appreciable amount of inclusion complex is made in which the complexant is an anionic surfactant. Independently, embodiments are contemplated in which no appreciable amount of inclusion complex is made in which the complexant is a surfactant.

Independently, embodiments are contemplated in which no appreciable amount of inclusion complex is made in which the complexant is a ketone with 3 or 4 carbon atoms. Independently, embodiments are contemplated in which no appreciable amount of inclusion complex is made in which the complexant is a ketone.

Independently, embodiments are contemplated in which no appreciable amount of inclusion complex is made in which the complexant is carbon disulfide.

In some embodiments (herein called "release embodiments"), an inclusion complex of the present invention is used in a way that releases a useful complexant in a situation that makes use of the useful property or properties of the useful complexant. In some release embodiments, the inclusion complex of the present invention is dried prior to release of the useful complexant.

Various cyclopropenes are known to have beneficial effects when used for treating plants or plant parts. Independently, in some release embodiments, a dried inclusion complex containing a cyclopropene may be added to water; the water may be agitated, bubbled, or otherwise treated to cause release of cyclopropene in proximity to plants or plant parts, optionally in an enclosed volume. For another example, an inclusion complex containing a cyclopropene may be added to water, optionally along with other ingredients such as, for example, adjuvants, and the composition thus formed may be brought into contact with plants or plant parts, for example by spraying or dipping.

In some release embodiments, once the inclusion complex of the present invention is made, no substantial amount of useful complexant is released until the useful complexant is put to use. That is, in such embodiments, the amount of useful complexant that remains in the inclusion complex until the useful complexant is put to use is, by weight based on the weight of inclusion complex, 50% or more; or 75% or more; or 90% or more; or 99% or more.

For example, the useful complexant is a cyclopropene (i.e., a cyclopropene or a mixture of cyclopropenes). In some release embodiments, for example, the intended use of the cyclopropene is to bring the cyclopropene into contact with plants or plant parts. In such embodiments, it is contemplated that no substantial amount of cyclopropene is released from the inclusion complex until the inclusion complex is in contact with or in proximity to the plants or plant parts intended to be contacted by the cyclopropene.

It is to be understood that for purposes of the present specification and claims that the range and ratio limits recited herein can be combined. For example, if ranges of 60 to 120 and 80 to 110 are recited for a particular parameter, it is understood that the ranges of 60 to 110 and 80 to 120 are also contemplated. As a further, independent, example, if a particular parameter is disclosed to have suitable minima of 1, 2, and 3, and if that parameter is disclosed to have suitable maxima of 9 and 10, then all the following ranges are contemplated: 1 to 9, 1 to 10, 2 to 9, 2 to 10, 3 to 9, and 3 to 10.

### EXAMPLES

### Comparative Example C1: Purified Cyclodextrin

Using methods taught by G. Schmid, in Comprehensive Supramolecular Chemistry, vol. 3, pp. 41-56 (1966), purified alpha-cyclodextrin could be made by performing the following steps:
(i) Potato starch or corn starch could be liquified with alpha-amylase, and a 30% by weight solution of liquified starch in solvent could be prepared, using a solvent that is water mixed with 1-decanol.
(ii) The enzyme alpha-CGTase from Kebsiella oxytoca could be added to the solution.
(iii) The resulting precipitated inclusion complex of cyclodextrin and decanol could be removed from the solution, washed, and dried to form a powder of the inclusion complex.
(iv) The 1-decanol could be removed from the inclusion complex by steam distillation or extraction.
(v) The resulting mixture could be concentrated by vacuum distillation, treated with active carbon, crystallized, filtered, and dried to form a powder of pure cyclodextrin.

### Comparative Example C2: Comparative Method of Forming Inclusion Complex

Using the methods taught in US 6,017,849, an inclusion complex could be formed by performing the following steps:
(vi) 2.9 kg of the pure cyclodextrin powder produced as in Comparative Example C 1 could be dissolved in 0.575 liter of a buffer solution (mixture of 0.2 M sodium acetate and 0.2 M acetic acid to give pH of 3 to 5).
(vii) 1-MCP in gaseous form could be bubbled through to buffer solution to produce inclusion complex, which precipitates.
(viii) The inclusion complex could be separated from the buffer solution by vacuum filtration.
(ix) The inclusion complex could then be dried to form a dry powder.

### Example 3: Formation of Inclusion Complex

An inclusion complex could be formed by performing the following steps:
(1) A solution of liquified potato starch or corn starch could be produced by liquification by alpha-amylase in water, using methods similar to step (i) of Comparative Example C 1 except that no decanol would be used.
(2) alpha-CGTase could be added to the solution.
(3) gaseous 1-MCP could be added to the solution by bubbling to produce precipitated inclusion complex of cyclodextrin and 1-MCP.
(4) The resulting precipitated inclusion complex could be removed from the solution, washed, and dried to form a powder of the inclusion complex.

The method of Example 3 produces pure, dry powder of inclusion complex while performing fewer steps than the method of Comparative Examples C1 and C2.

## Claims

1. A method of making at least one inclusion complex comprising a useful complexant and a cyclodextrin, wherein said method comprises the steps of
(a) making said cyclodextrin, and
(b) mixing said cyclodextrin with said useful complexant,
wherein said method is conducted without purifying said cyclodextrin, and wherein said useful complexant comprises at least one cyclopropene.

2. The method of claim 1, wherein said step (a) comprises reacting at least one starch with at least one cyclodextrin-producing enzyme.

3. The method of claim 1, wherein said method further comprises the step of precipitating said inclusion complex or allowing said complex to precipitate.

4. The method of claim 1, wherein said inclusion complex does not comprise any alkane, hydroxy-substituted alkane, or halogenated hydrocarbon.

5. The method of claim 1,
wherein said step (a) comprises reacting at least one starch with at least one cyclodextrin-producing enzyme to form said cyclodextrin, and
wherein said method further comprises the step ofprecipitating said inclusion complex or allowing said inclusion complex to precipitate.

6. The method of claim 1, wherein said method further comprises the steps of drying said inclusion complex; adding said dried inclusion complex to water; and agitating, bubbling or otherwise treating said water to cause release of said cyclopropene in proximity to plants or plant parts in an enclosed volume.

7. The method of claim 1, wherein said method further comprises adding said inclusion complex to water; and bringing the composition thus formed into contact with plants or plant parts.

8. A composition useful for manufacturing inclusion complexes, wherein said composition comprises water, at least one starch, at least one enzyme, and at least one useful complexant, wherein said useful complexant comprises at least one cyclopropene, wherein said starch and said enzyme are capable of forming a cyclodextrin that is capable of forming an inclusion complex that comprises said cyclodextrin and said useful complexant.

9. The composition of claim 8 wherein said cyclopropene comprises 1-MCP.

## Patentansprüche

1. Ein Verfahren zur Herstellung von mindestens einem Inklusionskomplex, einen nützlichen Komplexbildner und ein Cyclodextrin beinhaltend, wobei das Verfahren die folgenden Schritte beinhaltet:
(a) Herstellung des Cyclodextrins, und
(b) Mischen des Cyclodextrins mit dem nützlichen Komplexbildner,
wobei das Verfahren ohne Reinigen des Cyclodextrins ausgeführt wird, und wobei der nützliche Komplexbildner mindestens ein Cyclopropen beinhaltet.

2. Verfahren gemäß Anspruch 1, wobei Schritt (a) das Reagieren von mindestens einer Stärke mit mindestens einem Cyclodextrin produzierenden Enzym beinhaltet.

3. Verfahren gemäß Anspruch 1, wobei das Verfahren ferner den Schritt des Ausfällens des Inklusionskomplexes oder des Ermöglichens des Ausfällens des Komplexes beinhaltet.

4. Verfahren gemäß Anspruch 1, wobei der Inklusionskomplex kein Alkan, mit Hydroxy substituiertes Alkan oder Halogenkohlenwasserstoff beinhaltet.

5. Verfahren gemäß Anspruch 1,
wobei Schritt (a) das Reagierenlassen von mindestens einer Stärke mit mindestens einem Cyclodextrin produzierenden Enzym beinhaltet, um das Cyclodextrin zu bilden, und
wobei das Verfahren ferner den Schritt des Ausfällens des Inklusionskomplexes oder des Ermöglichens des Ausfällens des Komplexes beinhaltet.

6. Verfahren gemäß Anspruch 1, wobei das Verfahren ferner die Schritte des Trocknens des Inklusionskomplexes; des Hinzufügens des getrockneten Inklusionskomplexes zu Wasser; und des Rührens, Perlenlassens oder anderweitig Behandelns des Wassers, um die Freigabe des Cyclopropens nahe Pflanzen oder Pflanzenteilen in einem eingeschlossenen Volumen zu bewirken, beinhaltet.

7. Verfahren gemäß Anspruch 1, wobei das Verfahren ferner das Hinzufügen des Inklusionskomplexes zu Wasser beinhaltet; und das In-Kontakt-Bringen der so gebildeten Zusammensetzung mit Pflanzen oder Pflanzenteilen.

8. Eine Zusammensetzung, die zur Erzeugung von Inklusionskomplexen nützlich ist, wobei die Zusammensetzung Wasser, mindestens eine Stärke, mindestens ein Enzym und mindestens einen nützlichen Komplexbildner beinhaltet, wobei der nützliche Komplexbildner mindestens ein Cyclopropen beinhaltet, wobei die Stärke und das Enzym fähig sind, ein Cyclodextrin zu bilden, das fähig ist, einen Inklusionskomplex zu bilden, der das Cyclodextrin und den nützlichen Komplexbildner beinhaltet.

9. Zusammensetzung gemäß Anspruch 8, wobei das Cyclopropen 1-MCP beinhaltet.

## Revendications

1. Une méthode de réalisation d'au moins un complexe d'inclusion comprenant un complexant utile et une cyclodextrine, ladite méthode comprenant les étapes consistant
(a) à réaliser ladite cyclodextrine, et
(b) à mélanger ladite cyclodextrine avec ledit complexant utile,
ladite méthode étant mise en oeuvre sans purification de ladite cyclodextrine, et ledit complexant utile comprenant au moins un cyclopropène.

2. La méthode de la revendication 1, dans laquelle ladite étape (a) comprend le fait de faire réagir au moins un amidon avec au moins une enzyme productrice de cyclodextrine.

3. La méthode de la revendication 1, ladite méthode comprenant en outre l'étape consistant à faire précipiter ledit complexe d'inclusion ou à laisser ledit complexe précipiter.

4. La méthode de la revendication 1, dans laquelle ledit complexe d'inclusion ne comprend ni alcane, ni alcane substitué par un hydroxy, ni hydrocarbure halogéné.

5. La méthode de la revendication 1,
dans laquelle ladite étape (a) comprend le fait de faire réagir au moins un amidon avec au moins une enzyme productrice de cyclodextrine afin de former ladite cyclodextrine, et
ladite méthode comprenant en outre l'étape consistant à faire précipiter ledit complexe d'inclusion ou à laisser ledit complexe d'inclusion précipiter.

6. La méthode de la revendication 1, ladite méthode comprenant en outre les étapes consistant à faire sécher ledit complexe d'inclusion ; à ajouter ledit complexe d'inclusion séché à de l'eau ; et à agiter, à faire barboter ou à traiter autrement ladite eau afin de provoquer la libération dudit cyclopropène à proximité de plantes ou de parties de plantes dans un volume clos.

7. La méthode de la revendication 1, ladite méthode comprenant en outre le fait d'ajouter ledit complexe d'inclusion à de l'eau ; et d'amener la composition ainsi formée au contact de plantes ou de parties de plantes.

8. Une composition utile pour la fabrication de complexes d'inclusion, ladite composition comprenant de l'eau, au moins un amidon, au moins une enzyme, et au moins un complexant utile, ledit complexant utile comprenant au moins un cyclopropène, ledit amidon et ladite enzyme étant capables de former une cyclodextrine elle-même capable de former un complexe d'inclusion qui comprend ladite cyclodextrine et ledit complexant utile.

9. La composition de la revendication 8 dans laquelle ledit cyclopropène comprend du 1-MCP.
